# EUROPÄISCHE PATENTSCHRIFT

(11) **EP 2 300 075 B1**
(45) Veröffentlichungstag und Bekanntmachung des Hinweises auf die Patenterteilung: **08.07.2015**
(21) Anmeldenummer: 09761457.2
(22) Anmeldetag: 09.06.2009
(51) Int. Cl.: G01M 3/26, A61M 5/00, B65D 3/00, A61M 5/315, A61M 5/50, A61M 5/31

(54) **Vorrichtung zum Setzen eines Stopfens bei gleichzeitiger Prüfung einer bestimmungsgemässen Lageausrichtung des Stopfens**
Device for placing a stopper while simultaneously checking that the stopper is correctly positioned
Dispositif pour placer un bouchon tout en vérifiant en même temps le positionnement conforme du bouchon

(30) Priorität: 12.06.2008 DE 102008030038
(43) Veröffentlichungstag der Anmeldung: 30.03.2011
(60) Teilanmeldung: 13003857.3
(73) Patentinhaber: Arzneimittel GmbH Apotheker Vetter & Co. Ravensburg, 88212 Ravensburg (DE)
(72) Erfinder: GLUNZ, Alexander, 88214 Ravensburg (DE); SCHROFF, Arno, 88693 Deggenhausertal (DE)
(74) Vertreter: Gleiss, Alf-Olav
(86) Internationale Anmeldenummer: PCT/EP2009/004133
(87) Internationale Veröffentlichungsnummer: WO 2009/149894

(56) Entgegenhaltungen:
- WO-A-2008/012611
- WO-A1-2006/128564
- WO-A1-2007/024957
- DE-A1- 3 044 550
- DE-A1- 4 117 134
- DE-A1- 19 927 117
- GB-A- 1 419 764
- GB-A- 2 324 999
- US-A- 4 501 306
- US-A- 4 811 252
- US-A1- 2003 100 866
- US-A1- 2004 139 789
- US-A1- 2006 129 084
- US-A1- 2006 252 991
- US-A1- 2007 267 092
- US-A1- 2008 035 233

## Beschreibung

Die Erfindung betrifft eine Vorrichtung zum Setzen eines Stopfens bei gleichzeitiger Prüfung einer bestimmungsgemäßen Lageausrichtung des Stopfens gemäß Oberbegriff des Anspruchs 1, und ein Verfahren zum Setzen eines Stopfens bei gleichzeitiger Prüfung einer bestimmungsgemäßen Lageausrichtung des Stopfens gemäß Anspruch 6.

Vorrichtungen der genannten Art sind bekannt. Sie umfassen ein Halteelement, mit dem ein Hohlkörper, beispielsweise eine Spritze oder Karpule, fixiert werden kann. Der Hohlkörper weist dabei wenigstens eine erste und eine zweite Öffnung auf. Es ist ein Stopfensetzmittel vorgesehen, das dazu dient, in die zweite Öffnung des Hohlkörpers einen Stopfen einzubringen. Der Hohlkörper umfasst typischerweise einen zylindrischen Innenraum, in den der im Wesentlichen zylindrische Stopfen durch die zweite Öffnung eingebracht werden soll. Sind die Längsachsen des Hohlkörpers und des Stopfens bestimmungsgemäß zueinander ausgerichtet, liegt die im Wesentlichen zylindrische Außenfläche des Stopfens an der typischerweise zylindrischen Innenfläche des Hohlkörpers an, sodass eine Dichtwirkung entsteht, wobei die zweite Öffnung des Hohlkörpers durch den Stopfen dicht verschlossen ist. Beim Setzen des Stopfens kann allerdings der Fehler auftreten, dass das Stopfensetzmittel den Stopfen nicht in bestimmungsgemäßer axialer Ausrichtung in die zweite Öffnung des Hohlkörpers einbringt. Der im Wesentlichen zylindrische Stopfen ist dann bezüglich der Längsachse des Hohlkörpers um eine Achse verdreht, die senkrecht sowohl auf seiner Längsachse wie auch auf der Längsachse des Hohlkörpers steht. Dies hat zur Folge, dass die im Wesentlichen zylindrische Mantelfläche des Stopfens nicht oder nicht vollständig an der typischerweise zylindrischen Innenfläche des Hohlkörpers anliegt, sodass hier eine reduzierte oder gar keine Dichtwirkung gegeben ist. Weist also der Stopfen eine in diesem Sinne nicht bestimmungsgemäße Lageausrichtung auf, ist die zweite Öffnung des Hohlkörpers nicht dicht verschlossen. Es kann auch vorkommen, dass das Stopfensetzmittel aufgrund eines Fehlers keinen Stopfen in die zweite Öffnung des Hohlkörpers einbringt. Auch in diesem Fall ist selbstverständlich die zweite Öffnung des Hohlkörpers nicht dicht verschlossen.

Gewöhnlich wird nach dem Einbringen des Stopfens in die zweite Öffnung des Hohlkörpers durch die erste Öffnung desselben ein Medium, beispielsweise eine pharmazeutische Substanz, eingebracht. Der Stopfen soll dann unter anderem verhindern, dass dieses Medium durch die zweite Öffnung des Hohlkörpers austreten kann. Weist der Stopfen allerdings eine nicht bestimmungsgemäße Lageausrichtung auf oder ist gar kein Stopfen vorhanden, ist also die zweite Öffnung des Hohlkörpers nicht dicht verschlossen, kann das Medium hier austreten und beispielsweise eine Befüllungsanlage oder eine gesamte Fertigungsstraße, die zumindest eine Stopfensetz- und vorzugsweise eine Befüllungsstation umfasst, kontaminieren. Typischerweise sind die in den Hohlkörper einzubringenden Medien sehr teuer, sodass Verluste durch nicht bestimmungsgemäß ausgerichtete oder fehlende Stopfen vermieden werden sollen.

Um daher die Lageausrichtung beziehungsweise des Vorhandenseins des Stopfens in dem Hohlkörper prüfen zu können, umfassen bekannte Vorrichtungen typischerweise mindestens eine Kamera, die Bilder des Hohlkörpers an ein Auswertungssystem liefert. Dieses wertet die Bilder im Allgemeinen softwareunterstützt aus, um festzustellen, ob der Stopfen bestimmungsgemäß ausgerichtet wurde. Es sind auch Systeme bekannt, die Lichtleiter umfassen, wobei ein Lichtstrahl durch den Teil des Hohlkörpers geschickt wird, in dem bestimmungsgemäß der Stopfen angeordnet sein soll. Auf der dem Lichtleiter gegenüberliegenden Seite des-Hohlkörpers ist eine Lichtmesseinrichtung, beispielsweise eine Fotodiode, vorgesehen, die im Wesentlichen erfasst, ob ein Lichtstrahl den Hohlkörper durchsetzt. Auf diese Weise kann festgestellt werden, ob der Stopfen überhaupt in dem Hohlkörper angeordnet ist.

Kamerasysteme haben den Nachteil, sehr teuer zu sein und eine aufwendige und fehleranfällige Auswertesoftware zu brauchen. Allerdings kann mit ihnen die tatsächliche Ausrichtung eines Stopfens erfasst werden. Lichtleitersysteme sind demgegenüber sehr viel einfacher aufgebaut und dadurch kostengünstiger; sie brauchen auch keine besonders komplizierte Auswerte- oder Steuerungssoftware. Allerdings kann mit ihrer Hilfe lediglich erfasst werden, ob überhaupt ein Stopfen in den Hohlkörper eingebracht wurde. Die bestimmungsgemäße Lageausrichtung gegenüber dem Hohlkörper ist nicht ermittelbar, weil der zur Prüfung benutzte Lichtstrahl auch von einem Stopfen ausgeblendet werden kann, der um eine senkrecht auf seiner Längsachse stehende Achse verdreht in den Hohlkörper eingebracht wurde.

Aus der britischen Patentschrift GB 1 419 764 A geht eine Vorrichtung zum Testen der Dichtheit von Hohlkörpern hervor, die insbesondere eingerichtet ist zur Prüfung von Rohrkupplungen und Rohrfittings. Aus der US-amerikanischen Patentanmeldung US 2006/0129084 A1 geht ein Verfahren hervor, mithilfe dessen ein irregulärer Betrieb einer Spritzenpumpe festgestellt werden kann. Aus der deutschen Offenlegungsschrift DE 41 17 134 A1 geht ein Verfahren zur Dichtigkeitsprüfung von medizinischen Schlauch- und Katheter-Systemen hervor, bei dem vorgesehen ist, dass die Prüflinge während der Prüfung in Längsrichtung gezogen sind. Aus der US-amerikanischen Patentanmeldung US 2006/0252991 A1 gehen eine Vorrichtung und ein Verfahren für einen Integritätstest eines Endoskops hervor. Aus der US-amerikanischen Patentanmeldung US 2004/0139789 A1 ist ein Lecktester für ein Endoskop und ein hiermit verbundenes Verfahren bekannt.

Aus der internationalen Patentanmeldung mit der Veröffentlichungsnummer WO 2006/128564 A1 und aus der internationalen Patentanmeldung mit der Veröffentlichungsnummer WO 2007/024957 A1 gehen jeweils Verfahren hervor, mithilfe derer Leckagetests für Spritzen mit bereits gesetzten Stopfen möglich ist.

Aufgabe der vorliegenden Erfindung ist es daher, eine Vorrichtung zu schaffen, welche die genannten Nachteile nicht aufweist, um ein irrtümliches Befüllen von Hohlkörpern mit fehlerhaft oder gar nicht gesetzten Stopfen nach Möglichkeit zu vermeiden.

Die der Erfindung zugrundeliegende Aufgabe wird gelöst durch eine Vorrichtung mit den Merkmalen des Anspruchs 1. Die Vorrichtung weist einen Messkopf auf, der von mindestens einem Kanal durchsetzt wird, wobei der Kanal in Fluidverbindung mit der ersten Öffnung des Hohlkörpers bringbar ist. Weiterhin umfasst die Vorrichtung einen Druckaufnehmer, der in Fluidverbindung mit dem Kanal des Messkopfs bringbar ist. Auf diese Weise steht der Druckaufnehmer auch in Fluidverbindung mit der ersten Öffnung des Hohlkörpers. Ein im Innenraum des Hohlkörpers herrschender Druck ist daher durch den Druckaufnehmer erfassbar. Insbesondere ist ein Überdruck im Inneren des Hohlkörpers erfassbar, wodurch festgestellt werden kann, ob der Stopfen im Hohlkörper bestimmungsgemäß ausgerichtet ist. Wie bereits gesagt, verschließt ein bestimmungsgemäß ausgerichteter Stopfen die zweite Öffnung des Hohlkörpers. Wird der Stopfen von dem Stopfensetzmittel in die zweite Öffnung eingebracht, wird gleichzeitig das Gasvolumen im Innenraum des Hohlkörpers komprimiert. Auf diese Weise erhöht sich der Innendruck in dem Hohlkörper, wobei sich auch der Druck in dem mindestens einen Kanal des Messkopfs und damit auch im Bereich des Druckaufnehmers erhöht, weil diese Elemente in Fluidverbindung mit der ersten Öffnung des Hohlkörpers stehen. Beim Setzen des Stopfens durch das Stopfensetzmittel entsteht also ein durch den Druckaufnehmer messbarer Überdruck, wenn der Stopfen bestimmungsgemäß ausgerichtet ist. Weist der Stopfen dagegen keine bestimmungsgemäße Lageausrichtung auf, ist er also verdreht, kann er die zweite Öffnung des Hohlkörpers nicht dicht verschließen. Dementsprechend kann beim Setzen des Stopfens Luft aus dem Innenraum des Hohlkörpers durch die zweite Öffnung entweichen, sodass zumindest ein geringerer Überdruck an dem Druckaufnehmer erfassbar ist. Fehlt der Stopfen ganz, bleibt die zweite Öffnung des Hohlkörpers während eines Stopfensetztakts des Stopfensetzmittels vollständig offen, sodass kein erhöhter Druck an dem Druckaufnehmer erfassbar ist.

Bevorzugt wird eine Vorrichtung, die sich dadurch auszeichnet, dass eine Druckquelle vorgesehen ist, die in Fluidverbindung mit der ersten Öffnung des Hohlkörpers bringbar ist. Mit Hilfe der Druckquelle kann ein Medium, beispielsweise ein Inertgas oder Luft, in den Hohlkörper eingebracht werden. Es ist eine vorzugsweise externe Druckquelle vorgesehen, die das Medium -vorzugsweise ein bestimmtes Volumen des Mediums- bevorzugt durch den Kanal des Messkopfs in den Hohlkörper einbringen kann. Das durch die Druckquelle in den Hohlkörper eingebrachte Medium erhöht zusätzlich zu der Kompression aufgrund des Setzens des Stopfens den Innendruck in dem Hohlkörper, sodass ein Überdruck entsteht, der von der Ausrichtung des Stopfens abhängt. Auch hier wird regelmäßig ein höherer Überdruck erreicht werden, wenn sich der Stopfen in seiner bestimmungsgemäßen Lageausrichtung befindet, als wenn der Stopfen verdreht oder nicht vorhanden ist.

Weitere bevorzugte Ausführungsbeispiele ergeben sich aus den Unteransprüchen.

Es wird auch eine nicht zur Erfindung gehörende Vorrichtung zum Prüfen einer bestimmungsgemäßen Lageausrichtung eines Stopfens beschrieben, wobei die Prüfung im Anschluss an das Einbringen des Stopfens in den Hohlkörper in einem separaten Schritt und gegebenenfalls in einem getrennten Bereich der Fertigungsstraße durchgeführt werden kann. Auch in diesem Fall soll die bestimmungsgemäße Lageausrichtung des Stopfens vor dem Einbringen eines vorzugsweise pharmazeutischen Stoffes in den Hohlkörper geprüft werden.

Diese nicht zur Erfindung gehörende Vorrichtung umfasst ebenfalls ein Halteelement zur Fixierung eines Hohlkörpers, einen Messkopf, der von mindestens einem Kanal durchsetzt wird, wobei der Kanal in Fluidverbindung mit einer ersten Öffnung des Hohlkörpers bringbar ist. Dabei ist allerdings bereits ein Stopfen in eine zweite Öffnung des Hohlkörpers eingebracht, weil hier der Prüfschritt separat von dem Setzen des Stopfens erfolgen soll. Es ist auch ein Druckaufnehmer vorgesehen, der in Fluidverbindung mit dem Kanal des Messkopfs bringbar ist. Die Vorrichtung zeichnet sich dadurch aus, dass eine Druckquelle vorgesehen ist, die in Fluidverbindung mit der ersten Öffnung des Hohlkörpers bringbar ist. Durch die Druckquelle kann ein vorzugsweise vorgegebenes Volumen eines Mediums, beispielsweise eines Inertgases oder Luft, in den Innenraum des Hohlkörpers eingebracht werden, sodass dort ein an dem Druckaufnehmer erfassbarer Überdruck entsteht, wenn der Stopfen bestimmungsgemäß ausgerichtet ist und damit die zweite Öffnung des Hohlkörpers dicht verschließt. Ist der Stopfen dagegen nicht bestimmungsgemäß ausgerichtet, also verdreht, kann er die zweite Öffnung nicht dicht verschließen, sodass hier zumindest ein Teil des von der Druckquelle in den Hohlkörper eingebrachten Mediums entweichen kann. So bildet sich zumindest ein geringerer Überdruck aus als dies der Fall ist, wenn der Stopfen korrekt ausgerichtet ist. Gegebenenfalls ist es möglich, dass sich kein Überdruck ausbildet. Insbesondere wenn in dem vorangegangenen Stopfensetztakt kein Stopfen gesetzt wurde, kann sich auch kein Überdruck ausbilden, weil die zweite Öffnung des Hohlkörpers vollständig offen ist. Es ist also möglich, anhand des an dem Druckaufnehmer erfassten Drucks festzustellen, ob der Stopfen in dem Hohlkörper bestimmungsgemäß ausgerichtet ist, beziehungsweise ob überhaupt ein Stopfen vorhanden ist.

Durch die Erfassung des an dem Druckaufriehmer anliegenden Drucks sind also Gutteile, das heißt Hohlkörper mit korrekt ausgerichteten Stopfen, auf sehr einfache Art von Schlechtteilen, also Hohlkörpern mit verdreht oder gar nicht gesetzten Stopfen, unterscheidbar.

Bevorzugt wird eine Vorrichtung, bei der das Halteelement von dem Messkopf umfasst ist. Das Halteelement kann also an dem Messkopf angeordnet sein, es kann aber auch ein Teil des Messkopfs sein. Es ist auch möglich, dass der Messkopf insgesamt als Halteelement ausgebildet ist. Auf diese Weise ist eine besonders einfache Konstruktion der hier angesprochenen Vorrichtungen möglich.

Es wird auch eine Vorrichtung bevorzugt, bei der der Stopfen ein Endstopfen für eine Einkammerkarpule oder eine Einkammerspritze ist.

Auch bevorzugt wird eine Vorrichtung, bei der der Stopfen ein Mittelstopfen für eine Doppel- oder Mehrkammerkarpule oder eine Doppel- oder Mehrkammerspritze ist.

Weitere vorteilhafte Ausgestaltungen ergeben sich aus den Unteransprüchen.

Aufgabe der Erfindung ist es weiterhin, ein Verfahren zu schaffen, durch das ein Stopfen in einen Hohlkörper eingebracht werden kann, wobei gleichzeitig die bestimmungsgemäße Lageausrichtung des Stopfens geprüft werden kann.

Die Aufgabe wird gelöst durch ein Verfahren mit den Schritten, die in Anspruch 6 angegeben sind. Ein Hohlkörper wird mit Hilfe eines Halteelements gehalten. Es wird eine Fluidverbindung zwischen einem einen Messkopf durchsetzenden Kanal und einem Druckaufnehmer bereitgestellt. Außerdem wird eine Fluidverbindung zwischen dem Kanal und einer ersten Öffnung des Hohlkörpers bereitgestellt. Insgesamt ergibt sich so eine Fluidverbindung zwischen der ersten Öffnung des Hohlkörpers - also auch zwischen einem Innenraum des Hohlkörpers - und dem Druckaufnehmer. Es wird ein Stopfen in eine zweite Öffnung des Hohlkörpers eingeführt, wobei während des Einführens des Stopfens ein an dem Druckaufnehmer abgreifbarer Druck erfasst wird. Ist der Stopfen beim Einführen korrekt ausgerichtet, kann am Druckaufnehmer ein höherer Druck abgegriffen werden, als dies bei verdrehtem oder nicht vorhandenem Stopfen der Fall ist. Es ist also sehr einfach möglich, anhand der Druckmessung festzustellen, ob ein Stopfen vorhanden und korrekt ausgerichtet ist.

Bevorzugt wird ein Verfahren, bei dem zusätzlich eine Fluidverbindung zwischen einer Druckquelle und der ersten Öffnung des Hohlkörpers bereitgestellt wird. Dies geschieht vor der Einführung des Stopfens. Während der Einführung des Stopfens wird dann ein vorgegebenes Volumen eines Mediums, beispielsweise eines Inertgases oder Luft, in die erste Öffnung des Hohlkörpers mit Hilfe der Druckquelle eingebracht. Ein an dem Druckaufnehmer erfassbarer Überdruck entsteht hier also zum einen durch die Kompression des Volumens im Innenraum des Hohlkörpers und zum anderen durch das zusätzliche Einbringen eines vorgegebenen Volumens eines Mediums durch die Druckquelle, falls der Stopfen bestimmungsgemäß ausgerichtet ist. Ist der Stopfen dagegen verdreht oder gar nicht vorhanden, bildet sich ein kleinerer oder gegebenenfalls gar kein Überdruck aus, was wiederum an dem Druckaufnehmer erfassbar ist.

Es wird auch ein nicht zur Erfindung gehörendes Verfahren beschrieben, mit Hilfe dessen eine bestimmungsgemäße Lageausrichtung eines Stopfens geprüft werden kann, nachdem der Stopfen in einen Hohlkörper eingebracht wurde.

Ein Hohlkörper wird dabei mit Hilfe eines Halteelements gehalten. Es wird eine Fluidverbindung zwischen einem einen Messkopf durchsetzenden Kanal und einem Druckaufnehmer bereitgestellt. Außerdem wird eine Fluidverbindung zwischen dem Kanal und einer ersten Öffnung des Hohlkörpers bereitgestellt, sodass insgesamt eine Fluidverbindung zwischen der ersten Öffnung des Hohlkörpers - also auch einem Innenraum des Hohlkörpers -und dem Druckaufnehmer besteht. Bei diesem nicht zur Erfindung gehörenden Verfahren ist bereits ein Stopfen in eine zweite Öffnung des Hohlkörpers eingesetzt, weil die Prüfung nach dem Einbringen des Stopfens erfolgen soll. Es wird eine Fluidverbindung zwischen einer Druckquelle und der ersten Öffnung des Hohlkörpers bereitgestellt. Mit Hilfe der Druckquelle wird ein vorgegebenes Volumen eines Mediums, beispielsweise eines Inertgases oder Luft, in die erste Öffnung des Hohlkörpers eingebracht, während der an dem Druckaufnehmer abgreifbare Druck erfasst wird. Es ist auch in diesem Fall offensichtlich, dass sich bei korrekt ausgebildetem Stopfen ein höherer Überdruck in dem Innenraum des Hohlkörpers ausbilden kann, der dann auch an dem Druckaufnehmer erfassbar ist, weil dieser in Fluidverbindung mit dem Innenraum steht. Ist der Stopfen dagegen verdreht oder nicht vorhanden, kann sich nur ein geringerer oder gar kein Überdruck ausbilden, was auch mittels des Druckaufnehmers erfassbar ist. Über die Druckmessung kann also auch hier eine Aussage darüber getroffen werden, ob ein Stopfen in dem Hohlkörper vorhanden und bestimmungsgemäß ausgerichtet ist.

Vorteilhafte Ausgestaltungen ergeben sich aus den Unteransprüchen.

Die Erfindung wird im Folgenden anhand der Zeichnung näher erläutert.

Es zeigen:
- Figur 1: eine schematische Ansicht eines Teils der Vorrichtung gemäß Anspruch 1;
- Figur 2a: die Vorrichtung gemäß Anspruch 1 vor dem Einbringen des Stopfens in den Hohlkörper;
- Figur 2b: die Vorrichtung gemäß Anspruch 1 beim Einbringen des Stopfens in den Hohlkörper;
- Figur 2c: die Vorrichtung gemäß Anspruch 1 nach dem Einbringen des Stopfens in den Hohlkörper;
- Figur 3: die Vorrichtung gemäß Anspruch 1 beim Einbringen eines nicht bestimmungsgemäß ausgerichteten Stopfens in den Hohlkörper, und
- Figur 4: eine schematische Ansicht einer nicht zur Erfindung gehörenden Vorrichtung.

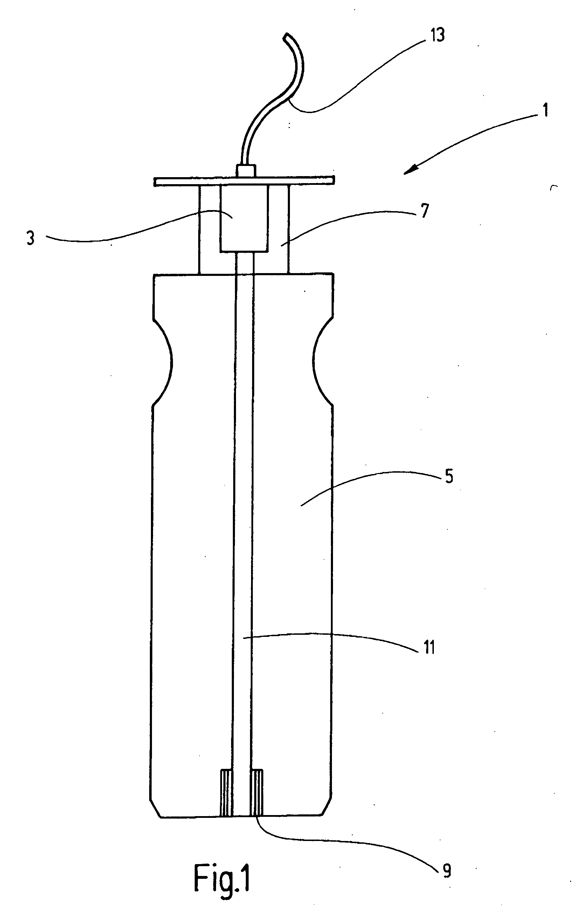

Figur 1 zeigt einen Teil einer Vorrichtung 1, mit Hilfe derer eine bestimmungsgemäße Lageausrichtung eines nicht dargestellten Stopfens in einem ebenfalls nicht dargestellten Hohlkörper geprüft werden kann.

Die Vorrichtung 1 umfasst einen Druckaufnehmer 3 und einen Messkopf 5. Der Druckaufnehmer 3 ist über einen Anschlussadapter 7 mit dem Messkopf 5 verbunden. Ein Anschlussadapter 7 ist dabei lediglich ein besonders einfach realisierbares Verbindungsmittel zwischen dem Druckaufnehmer 3 und dem Messkopf 5. Es ist auch möglich, an dem Messkopf 5 beispielsweise eine Olive vorzusehen, die mit einem Schlauch verbunden ist. Dieser Schlauch kann beispielsweise über eine weitere Olive mit dem Druckaufnehmer 3 in Verbindung stehen. Wesentlich ist nur, dass der Druckaufnehmer 3 mit dem Messkopf 5 in Fluidverbindung steht, sodass ein in dem Messkopf 5 vorliegender Druck auf den Druckaufnehmer 3 übertragbar ist.

An seinem -vom Betrachter aus gesehen- unteren Ende weist der Messkopf 5 vorzugsweise ein Dichtelement 9 auf, das einer druckdichten Fluidverbindung einer ersten Öffnung eines nicht dargestellten Hohlkörpers mit dem Messkopf 5 dient. Dabei weist der Hohlkörper typischerweise einen Kopf auf, der die erste Öffnung umfasst, und dessen Durchmesser kleiner ist als der Außendurchmesser des Dichtelements 9. Bei einer planen Anlage des Kopfes des Hohlkörpers an der -vom Betrachter aus gesehen- unteren Seite des Dichtelements 9 unter Aufbringung einer gewissen Kraft wird das Dich-telement 9 komprimiert und liegt so dichtend an dem Kopf des Hohlkörpers an. Ein Innenraum des Hohlkörpers steht dann über die erste Öffnung in dichter Fluidverbindung mit dem Messkopf 5.

Mindestens ein Kanal 11 durchsetzt den Messkopf 5 und gegebenenfalls auch den Anschlussadapter 7, sodass der Kanal 11 in Fluidverbindung mit der ersten Öffnung und damit auch mit dem Innenraum des Hohlkörpers, als auch in Fluidverbindung mit dem Druckaufnehmer 3 steht. Auf diese Weise ist ein im Innenraum des Hohlkörpers herrschender Überdruck durch den Druckaufnehmer 3 messbar. An diesem ist vorzugsweise ein Kabel 13 vorgesehen, mittels dessen ein den gemessenen Druck darstellendes Signal an eine nicht dargestellte Steuerungseinrichtung weitergegeben werden kann, in welcher der an dem Druckaufnehmer 3 anliegende Druck registrierbar ist.

Der Messkopf 5 ist bei dem dargestellten Ausführungsbeispiel als Halteelement ausgebildet, das den Hohlkörper zumindest in axialer Richtung fixiert, und in das die beim Einbringen des Stopfens entstehenden Kräfte einleitbar sind.

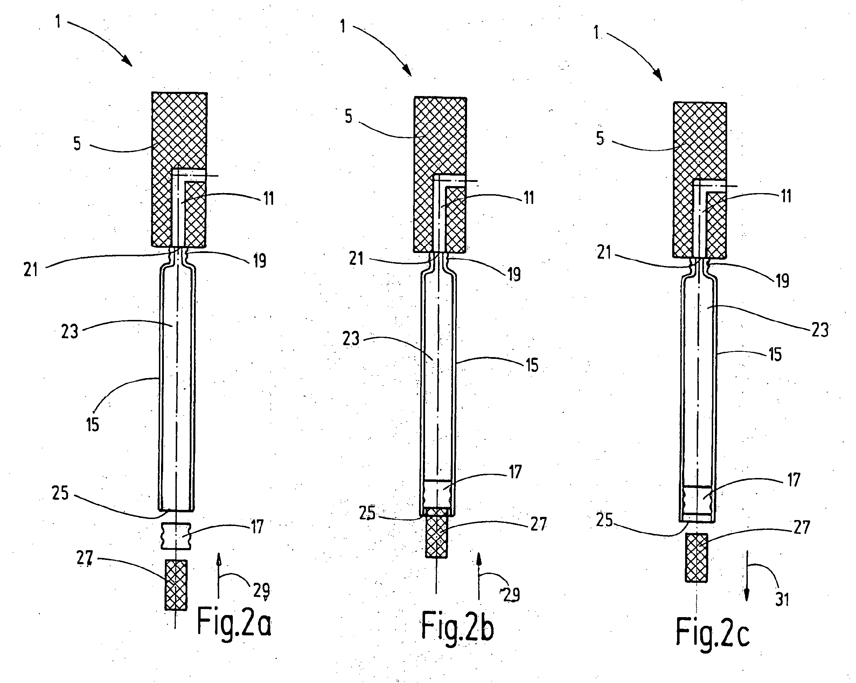

Figur 2 zeigt eine schematische Übersicht über das Setzen eines Stopfens mit Hilfe einer Vorrichtung 1. Gleiche und funktionsgleiche Elemente sind mit gleichen Bezugszeichen versehen, sodass insofern auf die vorangegangene Beschreibung verwiesen wird.

In Figur 2a ist der Messkopf 5 dargestellt, der hier von einem abgewinkelten Kanal 11 durchsetzt wird. Der Anschlussadapter 7 und der Druckaufnehmer 3 sind nicht dargestellt.

Die hier gewählte Darstellung ist rein schematisch. Es ist nicht not-wendig, dass der Druckaufnehmer 3 und der Messkopf 5 als separate Teile ausgebildet sind. Insbesondere können der Druckaufnehmer 3 und der Messkopf 5 integral ausgebildet sein, sodass vorzugsweise der Messkopf 5 den Druckaufnehmer 3 umfasst. Bei dem dargestellten Ausführungsbeispiel ist der Messkopf 5 als Halteelement ausgebildet, wobei er insbesondere als Widerlager für einen Hohlkörper 15 dient. Während des Einbringens eines Stopfens 17 liegt der Hohlkörper 15 mit seinem Kopf 19 plan an dem Messkopf 5, gegebenenfalls insbesondere an dem hier nicht dargestellten Dichtelement 9 an.

Aus Figur 2a wird klar, dass der den Messkopf 5 durchsetzende Kanal 11 in Fluidverbindung mit einer ersten Öffnung 21 des Hohlkörpers 15 steht. Dadurch steht er auch mit einem Innenraum 23 des Hohlkörpers 15 in Fluidverbindung. Da der nicht dargestellte Druckaufnehmer 3 in Fluidverbindung mit dem Kanal 11 des Messkopfs 5 steht, steht er vermittelt durch diesen auch mit der ersten Öffnung 21 beziehungsweise dem Innenraum 23 des Hohlkörpers 15 in Fluidverbindung. Ein in dem Innenraum 23 herrschender Druck ist also durch den Druckaufnehmer 3 erfassbar.

Der Hohlkörper 15 weist an seinem - in axialer Richtung gesehen-dem Kopf 19 beziehungsweise der ersten Öffnung 21 gegenüberliegenden Ende eine zweite Öffnung 25 auf. In diese ist der Stopfen 17 mit Hilfe eines Stopfensetzmittels 27 einbringbar, was hier durch einen Pfeil 29 angedeutet ist. Der Stopfen 17 wird also dem Hohlkörper 15 von der dem Messkopf 5 gegenüberliegenden Seite her zugeführt. Hierdurch werden Kräfte in den Hohlkörper 15 eingeleitet, die in Richtung des Messkopfs 5 wirken und von diesem aufgenommen werden.

Der Stopfen 17 kann vorzugsweise Vorsprünge an seiner Mantelfläche aufweisen, die beim Einführen des Stopfens 17 in den Hohlkörper 15 - in radialer Richtung gesehen - komprimiert werden und so zu einer verbesserten Dichtwirkung beitragen, andererseits aber auch die Reibung des Stopfens 17 an einer Innenwandung des Hohlkörpers 15 minimieren, weil zwischen ihnen - in axialer Richtung gesehen - Bereiche angeordnet sind, die nicht an der Innenwandung anliegen.

Die Funktionsweise der Vorrichtung gemäß Anspruch 1 und damit auch des Verfahrens gemäß Anspruch 9 werden im Folgenden anhand der Figuren 2b und 2c näher erläutert. Gleiche und funktionsgleiche Elemente sind mit gleichen Bezugszeichen versehen, sodass insofern auf die vorangegangene Beschreibung verwiesen wird.

Bei dem dargestellten Ausführungsbeispiel beziehungsweise der dargestellten Ausführungsform des Verfahrens wird der Hohlkörper 15 mit Hilfe des als Halteelement ausgebildeten Messkopfs 5 gehalten. Wie bereits gesagt, wird der Stopfen 17 durch das Stopfensetzmittel 27 in die zweite Öffnung 25 des Hohlkörpers 15 eingeführt. Beim Einführen des Stopfens 17 in den Hohlkörper 15 wird das von dem Innenraum 23 umfasste Medium, typischerweise Luft oder ein Inertgas, komprimiert, weil das ihm zur Verfügung stehende Volumen verkleinert wird. Hierdurch entsteht ein Überdruck, der über den Kanal 11 und gegebenenfalls den Anschlussadapter 7 an den Druckaufnehmer 3 übertragen wird. Dieser erfasst den Überdruck und erzeugt ein Signal, das vorzugsweise über das Kabel 13 an die nicht dargestellte Steuerungseinrichtung weitergegeben wird. Diese registriert den Druck, der an dem Druckaufnehmer 3 anliegt, während der Stopfen 17 in den Hohlkörper 15 eingebracht wird.

Figur 2c zeigt die Vorrichtung 1 unmittelbar nach dem Einführen des Stopfens 17 in den Hohlkörper 15. Gleiche und funktionsgleiche Elemente sind mit gleichen Bezugszeichen versehen, sodass insofern auf die vorangegangene Beschreibung verwiesen wird. Das Stopfensetzmittel 27 entfernt sich nun wieder in Richtung eines Pfeils 31 von dem Stopfen 17 beziehungsweise dem Hohlkörper 15, um den Hohlkörper 15 freizugeben.

Im Anschluss wird der Hohlkörper 15 mit dem gesetzten Stopfen 17 aus dem Bereich der Vorrichtung 1 entfernt, und es wird ein neuer Hohlkörper 15 mit noch nicht gesetztem Stopfen 17 in Wirkverbindung mit der Vorrichtung 1 gebracht. Die Positionierung der Stopfen 17 läuft also notwendigerweise getaktet ab.

Die Steuerungseinrichtung registriert auch einen an dem Druckaufnehmer 3 anliegenden Druck, während kein Stopfen 17 in einen Hohlkörper 15 eingebracht wird. Von der Steuerungseinrichtung werden also zwei Drücke während zweier Phasen des Maschinentaktes registriert: Vorzugsweise wird der Maximaldruck registriert, der beim Einbringen des Stopfens 17 in den Hohlkörper 15 entsteht, und es wird in einer zweiten Phase des Takts ein Druck registriert, der an dem Druckaufnehmer 3 anliegt, während kein Stopfen 17 in den Hohlkörper 15 eingebracht wird, vorzugsweise während kein Hohlkörper 15 in Wirkverbindung mit dem Messkopf 5 steht. Der Begriff Wirkverbindung spricht hier an, dass der Hohlkörper 15 mit seinem Kopf 19 an dem Messkopf 5 anliegt, wobei eine Fluidverbindung zwischen der ersten Öffnung 21 und dem Kanal 11 besteht.

In Zusammenhang mit den hier angesprochenen Fluidverbindungen ist für den Fachmann offensichtlich, dass diese bevorzugt dicht ausgebildet sein sollen. Andernfalls ist es nämlich nicht oder nur schwer möglich, an dem Druckaufnehmer 3 den Druck zu messen, der im Innenraum 23 des Hohlkörpers 15 herrscht. Andererseits schaden kleinere Undichtigkeiten nicht notwendigerweise, solange zumindest eine Veränderung des in dem Innenraum 23 herrschenden Drucks beim Einbringen des Stopfens 17 und/oder beim Einbringen eines vorgegebenen Volumens eines Mediums mit Hilfe einer Druckquelle messbar ist.

Vorzugsweise erfasst der Druckaufnehmer 3 den gesamten Druckverlauf zumindest während der Dauer des Prüftaktes, das heißt während des Einbringens eines Stopfens 17 und/oder während des Einbringens eines vorgegebenen Volumens eines Mediums mit Hilfe der Druckquelle in den Innenraum 23.

Dieser Druckverlauf wird dann zur Auswertung an die Steuerungseinrichtung weitergegeben; wahlweise über ein Kabel 13 oder vorzugsweise auch drahtlos, beispielsweise über eine Bluetooth- oder Infrarotschnittstelle.

Die Steuerungseinrichtung wertet dann den Druckverlauf aus, in dem beispielsweise der Maximaldruck bestimmt wird. Wurde der Druckverlauf eine ausreichende Zeit vor dem Prüftakt bereits registriert, kann auch ein Referenzdruck als Basislinie aus dem Verlauf bestimmt werden. Es ist also nicht unbedingt erforderlich, dass ein an dem Druckaufnehmer 3 anliegender Referenzdruck vor der Einführung des Stopfens 17 oder vor dem Einbringen eines vorzugsweise vorgegebenen Volumens eines Mediums separat in der Steuerungseinrichtung registriert wird und dass getrennt hiervon der Druck während des Einführens des Stopfens oder während des Einbringens des vorgegebenen Volumens eines Mediums in der Steuerungseinrichtung registriert wird, sondern die Steuerungseinrichtung kann über eine ausreichende Zeitspanne einen kompletten Druckverlauf aufzeichnen und hieraus sowohl den für die Prüfung relevanten Maximalwert als auch den Referenzwert als Basislinie bestimmen.

Die Steuerungseinrichtung bildet in einem bevorzugten Ausführungsbeispiel die Differenz zwischen dem maximalen Druck, der an dem Druckaufnehmer 3 anliegt, während ein Stopfen 17 in den Hohlkörper 15 eingebracht wird, und einem Druck, der an dem Druckaufnehmer 3 anliegt, während kein Stopfen 17 in den Hohlkörper 15 eingebracht wird, vorzugsweise während kein Hohlkörper 15 in Wirkverbindung mit dem Messkopf 5 steht. Sie vergleicht dann den ermittelten Differenzdruck mit einem voreingestellten Sollwert.

Der Sollwert ist zuvor so eingestellt worden, dass er auf die konkreten Bedingungen der Fertigungstrasse angepasst ist. Er hängt beispielsweise von der Geschwindigkeit des Stopfensetzvorgangs ab. Er ist auch vom Startzeitpunkt, insbesondere von der Phasenlage des Stopfensetzvorgangs innerhalb des Maschinentakts abhängig. Außerdem ist der Sollwert abhängig von dem Bereich, in dem der Stopfen 17 innerhalb des Hohlkörpers 15 positioniert wird. Wird der Stopfen 17 nämlich weiter in den Hohlkörper 15 hineingeschoben, resultiert daraus eine stärkere Kompression des in dem Hohlkörper 15 enthaltenen Mediums, als wenn der Stopfen weniger weit in den Hohlkörper 15 eingebracht wird. Weiterhin ist der Sollwert auch von der Stopfenform, insbesondere von der Längsausdehnung des Stopfens 17, von der Länge des Hohlkörpers 15, in den der Stopfen eingebracht werden soll, und von dem Durchmesser des Hohlkörpers 15, in den der Stopfen 17 eingebracht werden soll abhängig.

Die Abhängigkeit von der Geschwindigkeit des Stopfensetzvorgangs spricht an, dass bei einem schnelleren Setzen des Stopfens 17 eher ein überschießender Druckstoß am Druckaufnehmer 3 registriert werden kann, als wenn der Stopfen 17 langsamer in den Hohlkörper 15 eingeführt wird. Auch kann sich der Druck über möglicherweise bestehende Restundichtigkeiten bei einem langsamen Einführen des Stopfens 17 eher ausgleichen als bei einem raschen Stopfensetzvorgang.

Die Abhängigkeit von der Phasenlage des Stopfensetzens innerhalb des Maschinentakts spricht an, dass die Druckmessung phasenrichtig erfolgen muss, weil sonst möglicherweise irrelevante und völlig aussagelose Druckwerte registriert werden, die aufgrund einer falschen Phasenlage nicht das Setzen des Stopfens 17 reflektieren. So sollte möglichst der Druck als Maximaldruck registriert werden, der in dem Prüfsystem vorherrscht, wenn der Stopfen 17 gerade seine Endposition erreicht.

Die anderen genannten Abhängigkeiten sprechen unmittelbar die Volumendifferenz an, die beim Vorgang des Stopfensetzens entsteht. Es ist unmittelbar verständlich, dass eine größere Volumendifferenz zu einem höheren Überdruck führt, während eine kleinere Volumendifferenz einen kleineren Überdruck zur Folge hat.

Wichtig ist, dass der Sollwert so bestimmt wird, dass er auf die konkret in der Fertigungsstrasse vorliegenden Bedingungen angepasst ist. Fasst man die genannten Bedingungen allgemein unter dem Begriff der Prüfbedingungen zusammen, so muss der Sollwert also auf verschiedene Prüfbedingungen anpassbar sein.

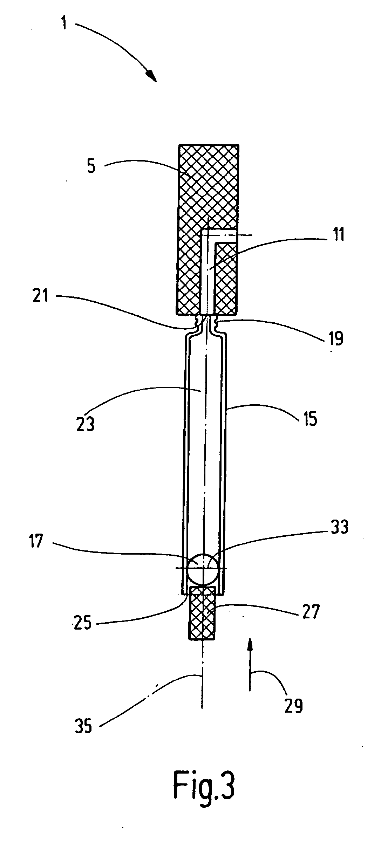

Figur 3 zeigt einen Ausschnitt des Stopfensetz- und Prüfvorgangs, bei dem, wie in Figur 2b, gerade ein Stopfen 17 in einen Hohlkörper 15 durch ein Stopfensetzmittel 27 eingeführt wird. Gleiche und funktionsgleiche Elemente sind mit gleichen Bezugszeichen versehen, sodass insofern auf die vorangegangene Beschreibung verwiesen wird.

Im Unterschied zu Figur 2b ist allerdings der Stopfen 17 in Figur 3 nicht bestimmungsgemäß ausgerichtet, sondern um eine Achse 33, die senkrecht auf seiner Längsachse und senkrecht auf der Längsachse 35 des Hohlkörpers 15 steht, verdreht, hier beispielhaft um 90°. In diesem Fall steht auch die Längsachse des Stopfens 17 senkrecht auf der Längsachse 35 des Hohlkörpers 15, während diese Achsen bei bestimmungsgemäßer Ausrichtung des Stopfens 17 parallel zueinander angeordnet sind.

Es ist offensichtlich, dass bei nicht bestimmungsgemäßer, also verdrehter Ausrichtung des Stopfens 17, dessen im Wesentlichen zylindrische äußere Mantelfläche nicht an einer Innenfläche des Hohlkörpers 15 anliegen kann.

Jedenfalls ist ein verdrehter Stopfen 17 nicht in der Lage, die zweite Öffnung 25 abzudichten, sodass beim Stopfensetzvorgang ein verringerter Druck an dem Druckaufnehmer 3 erfassbar ist. Fehlt der Stopfen 17 beim Stopfensetzvorgang vollständig, ist gar keine Druckerhöhung am Druckaufnehmer 3 erfassbar.

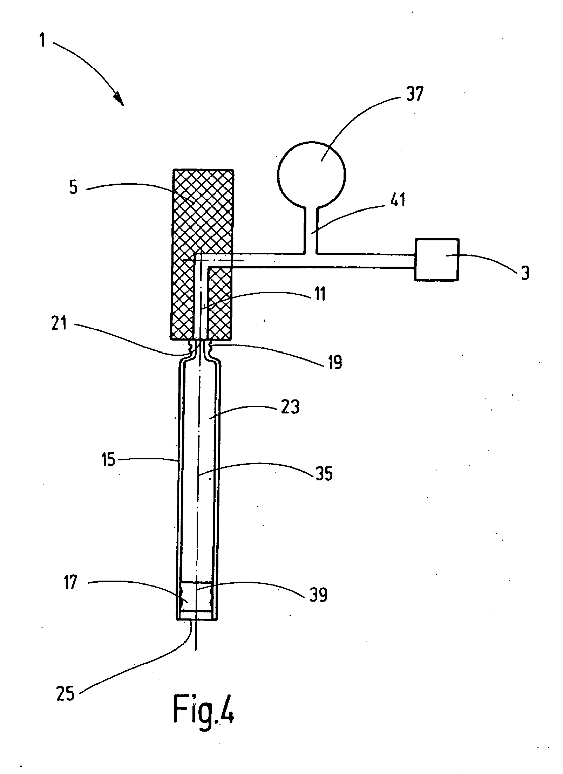

Im Folgenden soll ein nicht zur Erfindung gehörendes Beispiel einer Vorrichtung und eines Verfahrens in Zusammenhang mit Figur 4 näher erläutert werden. Gleiche und funktionsgleiche Elemente sind mit gleichen Bezugszeichen versehen, sodass insofern auf die vorangegangene Beschreibung verwiesen wird. In diesem Beispiel ist eine Druckquelle 37 vorgesehen, die ein Medium in den Hohlkörper 15 einbringen kann, weil sie in Fluidverbindung mit der ersten Öffnung 21 steht. Es kann beispielsweise eine Abzweigung von dem Kanal 11 vorgesehen sein, die mit der vorzugsweise externen Druckquelle 37 in Fluidverbindung steht. Auf diese Weise ist mittels der Druckquelle 37 ein vorzugsweise vorgegebenes Volumen eines Mediums, beispielsweise ein Inertgas oder Luft, in die erste Öffnung 21 und damit den Innenraum 23 des Hohlkörpers einbringbar, das genau dann zu einer Erhöhung des an dem Druckaufnehmer 3 anliegenden Drucks führt, wenn sich ein Stopfen 17 in dem Hohlkörper 15 befindet. Befindet sich umgekehrt kein Stopfen 17 in dem Hohlkörper 15, wird das in diesen eingeleitete Volumen unmittelbar an die Umgebung abgegeben, sodass keine Druckerhöhung stattfinden kann. Die Druckerhöhung fällt notwendigerweise höher aus, wenn der Stopfen 17 bestimmungsgemäß ausgerichtet und damit dichtend in dem Hohlkörper 15 angeordnet ist. Wurde der Stopfen stattdessen verdreht in den Hohlkörper eingebracht, sind Spalte oder undichte Stellen vorhanden, die den Aufbau eines entsprechend hohen Überdrucks verhindern.

Es ist offensichtlich, dass das in Figur 4 schematisch dargestellte, nicht zur Erfindung gehörende Beispiel einer Vorrichtung und eines Verfahrens einer Prüfung der bestimmungsgemäßen Ausrichtung des Stopfens 17 in dem Hohlkörper 15 nach dem Einbringen des Stopfens 17 in den Hohlkörper 15 dient. Es ist hier also ein separater Prüfschritt vorgesehen, der getrennt von dem Stopfensetztakt der Fertigungsstraße ausgeführt werden kann. Insbesondere kann dieser Prüfschritt auch räumlich von dem Stopfensetzschritt getrennt werden, sodass er also an einer separaten Vorrichtung der Fertigungsstraße erfolgt. Selbstverständlich ist es allerdings auch möglich, den Prüfschritt nur zeitlich nach dem Stopfensetztakt allerdings an derselben Vorrichtung 1 durchzuführen.

Es ist auch hier eine Steuereinrichtung vorgesehen, die der Erfassung eines Maximaldrucks dient, der dann entsteht, wenn ein vorzugsweise vorgegebenes Volumen eines Mediums durch die Druckquelle 37 in den Hohlkörper 15 eingebracht wird. Vorzugsweise ist die Steuerungseinrichtung auch in der Lage, einen Referenzdruck zu erfassen, der dann an dem Druckaufnehmer 3 anliegt, wenn kein Volumen eines Mediums aufgrund der Druckquelle 37 in den Hohlkörper 15 eingebracht wird, vorzugsweise wenn kein Hohlkörper 15 in Wirkverbindung mit dem Messkopf 5 steht.

In dem in Figur 4 dargestellten Beispiel ist der Stopfen 17 bestimmungsgemäß ausgerichtet, seine Längsachse 39 ist also parallel zu der Längsachse 35 des Hohlkörpers 15 orientiert.

Bezüglich der Steuerungseinrichtung beziehungsweise der Druckmessung und Druckregistrierung gilt auch in diesem Beispiel alles, was bereits in Zusammenhang mit Figur 2 gesagt wurde. Insbesondere kann die Steuerungseinrichtung auch hier einen kompletten Druckverlauf an dem Druckaufnehmer erfassen und aus diesem vorzugsweise einen Maximaldruck und gegebenenfalls auch einen Referenzdruck als Basislinie bestimmen.

Die Steuerungseinrichtung kann wahlweise den Maximaldruck mit einem Sollwert oder die Differenz aus dem registrierten Maximaldruck und dem Referenzdruck mit einem entsprechenden anderen Sollwert vergleichen. Auch dieser Sollwert hängt, wie oben beschrieben, von verschiedenen Prüfbedingungen ab und ist vorzugsweise an diese anpassbar.

Das vorliegende Beispiel soll für eine separate Prüfung für Hohlkörper 15 mit bereits gesetztem Stopfen 17 eingesetzt werden. In diesem Fall hängt der entstehende Maximaldruck beziehungsweise Referenzdruck im Wesentlichen von dem Volumen ab, das der Hohlkörper 15, der Kanal 11, gegebenenfalls der nicht dargestellte Anschlussadapter 7 beziehungsweise ein entsprechender Verbindungsschlauch oder ein anderes Verbindungsmittel und der Druckaufnehmer 3, sowie ein gegebenenfalls mit der Druckquelle 37 verbundener zusätzlicher Kanal oder Kanalabschnitt 41 bilden. Weiterhin hängt der Sollwert ab von dem Volumen des Mediums, das durch die Druckquelle 37 in den Hohlkörper 15 eingebracht wird.

Es ist bei einem anderen bevorzugten Ausführungsbeispiel der Vorrichtung und des Verfahrens auch möglich, die in Figur 2 gezeigte Vorrichtung durch eine Druckquelle 37 zu ergänzen. Diese wird dann ebenfalls in Fluidverbindung mit der ersten Öffnung 21 des Hohlkörpers 15 gebracht. So ist es möglich, ein vorzugsweise vorgegebenes Volumen eines Mediums, beispielsweise ein Inertgas oder Luft, in die erste Öffnung 21 des Hohlkörpers 15 und damit in dessen Innenraum 23 mit Hilfe der Druckquelle 37 einzubringen, während der Stopfen 17 durch das Stopfensetzmittel 27 in die zweite Öffnung 25 des Hohlkörpers 15 eingeführt wird. In diesem Fall kann an dem Druckaufnehmer 3 bei generell vorhandenem Stopfen 17 ein Druckanstieg registriert werden, der einerseits auf die Kompression des in dem Innenraum 23 vorliegenden Mediums durch das Einbringen des Stopfens 17 und andererseits auf das Einbringen des vorzugsweise vorgegebenen Volumens eines weiteren oder des gleichen Mediums durch die Druckquelle 37 zurückzuführen ist. Ist der Stopfen 17 nicht bestimmungsgemäß ausgerichtet sondern verdreht, fällt dieser Druckanstieg geringer aus, als es bei bestimmungsgemäß ausgerichtetem Stopfen 17 der Fall ist. Ist gegebenenfalls gar kein Stopfen 17 vorhanden, sollte je nach geometrischen Gegebenheiten, beispielsweise der Länge des Hohlkörpers 15 und dem Durchmesser der zweiten Öffnung 25 nur ein sehr geringer oder gar kein Druckanstieg an dem Druckaufnehmer 3 erfassbar sein. Die Auswertung des Druckanstiegs beziehungsweise Druckverlaufs oder einzelner während des Vorgangs erfasster Drücke kann wiederum in der bereits beschriebenen Weise in der Steuerungseinrichtung erfolgen. Selbstverständlich muss der Prüfung hier wiederum ein anderer Sollwert zugrunde gelegt werden, der an die konkreten Prüfbedingungen angepasst ist.

Allen Ausführungsbeispielen und Ausführungsformen ist gemein, dass ein von der Steuerungseinrichtung registrierter Druck mit einem Sollwert verglichen wird. Erreicht er den Sollwert oder fällt zumindest in einen vorgegebenen Toleranzbereich, handelt es sich bei dem Prüfteil offensichtlich um einen Hohlkörper 15 mit bestimmungsgemäß gesetztem Stopfen 17, also um ein Gutteil. Bleibt dagegen der registrierte Druck erheblich unter dem Sollwert, ist offensichtlich der Stopfen 17 verdreht oder gar nicht gesetzt. Es handelt sich bei dem Prüfling also um ein Schlechtteil.

Typischerweise erfolgt sowohl das Setzen des Stopfens 17 als auch das Prüfen von dessen bestimmungsgemäßer Ausrichtung in einer Fertigungsstraße. Diese umfasst verschiedene Stationen, unter anderem eine Stopfensetzstation und eine entweder von dieser umfasste oder separat angeordnete Prüfstation für die Prüfung der bestimmungsgemäßen Ausrichtung des Stopfens 17. In dieselbe Fertigungsstraße kann vorzugsweise eine Station integriert sein, in der ein Medium, beispielsweise eine pharmazeutische Substanz, in den Innenraum 23 des Hohlkörpers 15 eingebracht wird. Diese Substanz ist typischerweise teurer als der Hohlkörper 15 und der Stopfen 17. Deswegen muss darauf geachtet werden, dass keine Verluste der Substanz auftreten, weil ein Stopfen 17 nicht bestimmungsgemäß ausgerichtet und der Hohlkörper 15 aus diesem Grund nicht dicht verschlossen ist. Weiterhin kann das Austreten einer solchen Substanz durch die nicht dicht verschlossene zweite Öffnung 25 dazu führen, dass gegebenenfalls die gesamte Fertigungsstraße kontaminiert wird. Es können sich hier insbesondere mikrobiologische beziehungsweise hygienische Probleme ergeben, die schlimmstenfalls zu einem Totalausfall der Fertigungsstraße führen können, wobei eine aufwendige und teure Dekontamination und Sterilisation notwendig sein kann.

Es muss also vermieden werden, dass in Schlechtteile, also in Hohlkörper 15 mit nicht bestimmungsgemäß ausgerichtetem oder fehlendem Stopfen 17, die entsprechende Substanz eingebracht wird.

Hierzu ist ein Steuerungsprogramm vorzugsweise in der Steuerungseinrichtung vorgesehen, in dem intern eine Markierung setzbar ist, die der Erfassung eines Schlechtteils dient. Die Markierung kann vorzugsweise als virtuelle Marke in dem Steuerungsprogramm gesetzt werden. In einem anderen Ausführungsbeispiel ist es aber auch möglich, die Markierung als physikalische Kennzeichnung, beispielsweise als Aufkleber oder in sonstiger Art, an dem Schlechtteil anzubringen. Mit Hilfe der Markierung kann genau bestimmt werden, welche Teile in der Fertigungsstraße Schlechtteile sind. Die Markierung kann dazu verwendet werden, Schlechtteile sofort auszusondern, oder sie kann über die gesamte Fertigungsstraße weitergegeben werden, bis an deren Ende die Hohlkörper 15 mit nicht bestimmungsgemäß ausgerichteten Stopfen 17, die als Schlechtteile gekennzeichnet sind, als Ausschuss aussortiert werden. Insbesondere wird die Markierung als Schlechtteil an die Station innerhalb der Fertigungsstraße weitergereicht, die dazu dient, einen Hohlkörper 15 mit einem Medium, vorzugsweise einer pharmazeutischen Substanz zu füllen. Es kann so vermieden werden, dass Hohlkörper mit nicht bestimmungsgemäß ausgerichtetem oder gar nicht gesetztem Stopfen 17 mit der Substanzbefüllt werden. Hierdurch wird zum Einen der Verlust von zum Teil sehr teurer pharmazeutischer Substanz vermieden, zum Anderen wird vermieden, dass die pharmazeutische Substanz die Fertigungsstraße verschmutzt beziehungsweise kontaminiert. Insbesondere lassen sich so auch aufwendige Wartungsarbeiten wie beispielsweise eine Dekontamination beziehungsweise Sterilisation reduzieren oder sogar ganz vermeiden.

In allen beschriebenen Ausführungsbeispielen beziehungsweise Ausführungsformen sind die Vorrichtung und das Verfahren geeignet, die bestimmungsgemäße Ausrichtung eines Stopfens 17 in einem Hohlkörper 15 zu prüfen, wenn dieser Stopfen 17 das erste dichtende Element ist, das in die zweite Öffnung 25 des Hohlkörpers 15 eingebracht wird. Daraus ergibt sich, dass es sich bei dem Stopfen 17 um einen Endstopfen für eine Einkammerkarpule oder einer Einkammerspritze handeln kann. Es ergibt sich aber auch, dass der Stopfen 17 ein Mittelstopfen für eine Mehrkammerkarpule, eine Doppelkammerkarpule oder eine Mehrkammer- beziehungsweise Doppelkammerspritze sein kann. Diese Aufzählung ist beispielhaft und nicht einschränkend zu verstehen. Wesentlich ist, dass grundsätzlich die bestimmungsgemäße Ausrichtung des Stopfens 17 in dem Hohlkörper 15 prüfbar ist, so lange der Stopfen 17 das erste dichtende Element ist, das in den Hohlkörper 15 durch die zweite Öffnung 25 eingebracht wird. Auf diese Weise entsteht im Innenraum 23 des Hohlkörpers 15 beim Einbringen des Stopfens 17 ein Überdruck, der von dem Druckaufnehmer 3 durch die dem Stopfen 17 gegenüberliegende erste Öffnung 21 registriert werden kann. Andererseits kann durch die erste Öffnung 21 auch ein Druck in den Hohlkörper eingebracht werden, der ebenfalls durch den Druckaufnehmer 3 erfassbar ist.

Nach allem zeigt sich, dass alle Ausführungsbeispiele beziehungsweise Ausführungsformen der vorgeschlagenen Vorrichtung und des vorgeschlagenen Verfahrens auf sehr einfache, wenig fehleranfällige Weise eine rasche, sichere und gut reproduzierbare Prüfung der bestimmungsgemäßen Ausrichtung eines Stopfens 17 in einem Hohlkörper 15 erlauben. Insbesondere brauchen die vorgeschlagenen Vorrichtungen weder teure und komplizierte Kamerasysteme sowie ebenfalls teure und komplizierte Bildauswertungssoftware, sie sind aber trotzdem in der Lage, nicht nur zu detektieren, ob überhaupt ein Stopfen 17 gesetzt wurde, sondern sehr wohl auch zu prüfen, ob sich der Stopfen 17 in einer bestimmungsgemäßen Ausrichtung befindet.

## Patentansprüche

1. Vorrichtung zum Setzen eines Stopfens (17) in eine Spritze oder Karpule bei gleichzeitiger Prüfung einer bestimmungsgemäßen Lageausrichtung des Stopfens (17), mit
- einem Halteelement zur Fixierung eines Hohlkörpers (15) der Spritze oder Karpule, der zumindest eine erste und eine zweite Öffnung (21,25) aufweist;
- einem Stopfensetzmittel (27), das so ausgebildet ist, dass der Stopfen (17) mithilfe des Stopfensetzmittels (27) in die zweite Öffnung (25) des Hohlkörpers (15) einbringbar ist; unit
- einem von mindestens einem Kanal (11) durchsetzten Messkopf (5), wobei der Kanal (11) so ausgebildet ist, dass er in Fluidverbindung mit der ersten Öffnung (21) des Hohlkörpers-(15) bringbar ist, und mit
- einem Druckaufnehmer (3), der in Fluidverbindung mit dem Kanal (11) des Messkopfs (5) ist.

2. Vorrichtung nach Anspruch 1, **gekennzeichnet durch**, eine Druckquelle (37), die so ausgebildet ist, dass sie in Fluidverbindung mit der ersten Öffnung (21) des Hohlkörpers (15) bringbar ist.

3. Vorrichtung nach Anspruch 1 oder 2, **dadurch gekennzeichnet, dass** der Messkopf (5) und der Druckaufnehmer (3) integral ausgebildet sind.

4. Vorrichtung nach einem der vorhergehenden Ansprüche, **dadurch gekennzeichnet, dass** das Halteelement von dem Messkopf (5) umfasst ist.

5. Vorrichtung nach einem der vorhergehenden Ansprüche, **gekennzeichnet durch** eine Steuerungseinrichtung, die so ausgebildet ist, dass in ihr der an dem Druckaufnehmer (3) anliegende Druck registrierbar ist.

6. Verfahren zum Setzen eines Stopfens (17) in eine Spritze oder Karpule bei gleichzeitiger Prüfung einer bestimmungsgemäßen Lageausrichtung des Stopfens (17) in der Spritze oder Karpule, **gekennzeichnet durch** die folgenden Schritte:
- Halten eines Hohlkörpers (15) mit Hilfe eines Halteelements;
- Bereitstellen jeweils einer Fluidverbindung zwischen einem einen Messkopf (5) durchsetzenden Kanal (11) und einem Druckaufnehmer (3) einerseits und dem Kanal (11) und einer ersten Öffnung (21) des Hohlkörpers (15) andererseits;
- Einführen eines Stopfens (17) in eine zweite Öffnung (25) des Hohlkörpers (15), und
- Erfassen des an dem Druckaufnehmer (3) abgreifbaren Drucks während des Einführens des Stopfens (17).

7. Verfahren nach Anspruch 6, **gekennzeichnet durch** die zusätzlichen Schritte:
- Bereitstellen einer Fluidverbindung zwischen einer Druckquelle (37) und der ersten Öffnung (21) des Hohlkörpers (15) vor der Einführung des Stopfens (17), und
- Einbringen eines vorgegebenen Volumens eines Mediums in die erste Öffnung (21) des Hohlkörpers (15) mit Hilfe der Druckquelle (37) während des Einführens eines Stopfens (17).

8. Verfahren nach Anspruch 6, **dadurch gekennzeichnet, dass** an dem Druckaufnehmer (3) erfasste Druckwerte von einer Steuerungseinrichtung registriert werden.

9. Verfahren nach Anspruch 6, **dadurch gekennzeichnet, dass** ein als Einkammerkarpule oder -spritze ausgebildeter Hohlkörper verwendet wird, wobei als Stopfen (17) ein Endstopfen in die zweite Öffnung (25) eingeführt wird.

10. Verfahren nach Anspruch 6, **dadurch gekennzeichnet, dass** ein als Doppel- oder Mehrkammerkarpule oder-spritze ausgebildeter Hohlkörper verwendet wird, wobei als Stopfen (17) ein Mittelstopfen in die zweite Öffnung (25) eingeführt wird.

11. Verfahren nach den Ansprüchen 6 und 8, **gekennzeichnet durch** die folgenden Schritte:
- Erfassen eines an dem Druckaufnehmer (3) anliegenden Referenzdrucks vor der Einführung des Stopfens (17) und Registrierung dieses Drucks in der Steuerungseinrichtung;
- Registrieren des an dem Druckaufnehmer (3) erfassen Drucks während des Einführens des Stopfens (17) in der Steuerungseinrichtung;
- Bilden eines Differenzdrucks aus dem während des Einführens des Stopfens (17) gemessenen Druck und dem vor dem Einführen des Stopfens (17) gemessenen Referenzdrucks in der Steuerungseinrichtung, und
- Vergleichen des Differenzdrucks mit einem Sollwert.

12. Verfahren nach Anspruch 11, **dadurch gekennzeichnet, dass** der Sollwert auf verschiedene Prüfbedingungen angepasst wird.

13. Verfahren nach einem der vorhergehenden Ansprüche 6 bis 12. **dadurch gekennzeichnet, dass** der Hohlkörper (15) als Schlechtteil gekennzeichnet wird, wenn der Stopfen (17) nicht bestimmungsgemäß ausgerichtet ist.

## Claims

1. Device for placing a plug (17) into a syringe or carpule while simultaneously checking a correct position alignment of the plug (17), with
- a holding element for fixing a hollow body (15) of a syringe or carpule, which has at least one first and one second opening (21, 25);
- a plug placement means (27) being designed such that the plug (17) can be inserted into the second opening (25) of the hollow body (15) by means of the plug placement means (27); with
- a measuring head (5) that is traversed by at least one channel (11), the channel (11) being designed such that it can be brought into fluid connection with the first opening (21) of the hollow body (15), and with
- a pressure sensor (3), which is in fluid connection with the channel (11) of the measuring head (5).

2. Device according to claim 1, **characterized by** a pressure source (37) being designed such that it can be brought into fluid connection with the first opening (21) of the hollow body (15).

3. Device according to claim 1 or 2, **characterized in that** the measuring head (5) and the pressure sensor (3) are embodied in an integral manner.

4. Device according to one of the preceding claims, **characterized in that** the holding element is comprised by the measuring head (5).

5. Device according to one of the preceding claims, **characterized by** a control device being designed such that, in its interior, the pressure prevailing at the pressure sensor (3) can be registered.

6. Method for placing a plug (17) into a syringe or carpule while simultaneously checking a correct position alignment of the plug (17) within the syringe or carpule, **characterized by** the following steps:
- holding a hollow body (15) with the aid of a holding element;
- providing respectively one fluid connection between a channel (11) traversing a measuring head (5) and a pressure sensor (3) on the one hand and the channel (11) and a first opening (21) of the hollow body (15) on the other hand;
- inserting a plug (17) into a second opening (25) of the hollow body (15), and
- detecting the pressure detectable at the pressure sensor (3) during the insertion of the plug (17).

7. Method according to claim 6, **characterized by** the additional steps:
- providing a fluid connection between a pressure source (37) and the first opening (21) of the hollow body (15) before the insertion of the plug (17), and
- introducing a predetermined volume of a medium into the first opening (21) of the hollow body (15) with the aid of the pressure source (37) during the insertion of a plug (17).

8. Method according to claim 6, **characterized in that** pressure values detected at the pressure sensor (3) are registered by a control device.

9. Method according to claim 6, **characterized in that** a hollow body embodied as a single-chamber carpule or syringe is used, wherein an end plug is inserted as plug (17) into the second opening (25).

10. Method according to claim 6, **characterized in that** a hollow body embodied as a dual-chamber or multi-chamber carpule or a dual-chamber or multi-chamber syringe is used, wherein a central plug is inserted as plug (17) into the second opening (25).

11. Method according to claims 6 and 8, **characterized by** the following steps:
- detecting a reference pressure prevailing at the pressure sensor (3) before the insertion of the plug (17) and registering this pressure in the control device;
- registering the pressure detected at the pressure sensor (3) during the insertion of the plug (17) in the control device;
- forming a differential pressure from the pressure measured during the insertion of the plug (17) and the reference pressure measured before the insertion of the plug (17) in the control device, and
- comparing the differential pressure with a desired value.

12. Method according to claim 11, **characterized in that** the desired value is adapted to different test conditions.

13. Method according to one of the preceding claims 6 through 12, **characterized in that** the hollow body (15) is marked as a rejected part when the plug (17) is not aligned correctly.

## Revendications

1. Dispositif pour positionner un bouchon (17) dans une seringue ou carpule en simultanément vérifiant l'alignement conforme à sa destination du bouchon (17), avec
- un élément de retenue pour la fixation d'un corps creux (15) de la seringue ou carpule qui présent au moins une première et une seconde ouverture (21, 25) ;
- un moyen de positionnement du bouchon (27) configuré de telle manière que le bouchon (17) peut être inséré dans la seconde ouverture (25) du corps creux (15) à l'aide du moyen de positionnement du bouchon (27) ; avec
- une tête de mesure (5) traversée par au moins un conduit (11), le conduit (11) étant configuré de telle manière qu'il peut être mis en communication de fluide avec la première ouverture (21) du corps creux (15), et avec
- un capteur de pression (3) en communication de fluide avec le conduit (11) de la tête de mesure (5).

2. Dispositif selon la revendication 1, **caractérisé par** une source de pression (37) qui est configurée de telle manière qu'elle peut être mise en communication de fluide avec la première ouverture (21) du corps creux (15).

3. Dispositif selon l'une quelconque des revendications 1 ou 2, **caractérisé en ce que** la tête de mesure (5) et le capteur de pression (3) sont formés de manière intégrale.

4. Dispositif selon l'une quelconque des revendications précédentes, **caractérisé en ce que** l'élément de retenue est compris dans la tête de mesure (5).

5. Dispositif selon l'une quelconque des revendications précédentes, **caractérisé par** un dispositif de commande qui est configuré de telle manière qu'il peut enregistrer à l'intérieur la pression présente au capteur de pression (3).

6. Procédé pour positionner un bouchon (17) dans une seringue ou carpule en simultanément vérifiant l'alignement conforme à sa destination du bouchon (17) dans la seringue ou carpule, **caractérisé par** les étapes suivantes :
- tenir un corps creux (15) à l'aide d'un élément de retenue ;
- fournir une communication de fluide, en chaque cas, entre un conduit (11) traversant une tête de mesure (5) et un capteur de pression (3) d'une part, et le conduit (11) et une première ouverture (21) du corps creux (15) d'autre part ;
- insérer un bouchon (17) dans une seconde ouverture (25) du corps creux (15), et
- détecter la pression qui peut être prélevée au capteur de pression (3) pendant l'insertion du bouchon (17).

7. Procédé selon la revendication 6, **caractérisé par** les étapes additionnelles :
- fournir une communication de fluide entre une source de pression (37) et la première ouverture (21) du corps creux (15) avant l'insertion du bouchon (17), et
- introduire une volume prédéterminée d'un milieu dans la première ouverture (21) du corps creux (15) à l'aide de la source de pression (37) pendant l'insertion du bouchon (17).

8. Procédé selon la revendication 6, **caractérisé en ce que** les valeurs de pression détectées au capteur de pression (3) sont enregistrées d'un dispositif de commande.

9. Procédé selon la revendication 6, **caractérisé en ce qu'**un corps creux configuré comme carpule ou seringue à chambre unique est utilisé, un bouchon terminal étant inséré dans la seconde ouverture (25) comme bouchon (17).

10. Procédé selon la revendication 6, **caractérisé en ce qu'**un corps creux configuré comme carpule ou seringue à chambre double ou multiple est utilisé, un bouchon central étant inséré dans la seconde ouverture (25) comme bouchon (17).

11. Procédé selon les revendications 6 et 8, **caractérisé par** les étapes suivantes :
- détecter une pression de référence présente au capteur de pression (3) avant l'insertion du bouchon (17), et enregistrer cette pression dans le dispositif de commande ;
- enregistrer la pression détectée au capteur de pression (3) pendant l'insertion du bouchon (17) dans le dispositif de commande ;
- former, dans le dispositif de commande, une pression différentielle de la pression mesurée pendant l'insertion du bouchon (17) et la pression de référence mesurée avant l'insertion du bouchon (17) ; et
- comparer la pression différentielle avec une valeur de consigne.

12. Procédé selon la revendication 11, **caractérisé en ce que** la valeur de consigne est adaptée à des conditions d'essai différentes.

13. Procédé selon l'une quelconque des revendications précédentes 6 à 12, **caractérisé en ce que** le corps creux (15) est marqué comme rejet si le bouchon (17) n'est pas aligné conformément à sa destination.
